# DEMANDE DE BREVET EUROPEEN

(11) **EP 4 012 810 A1**
(43) Date de publication de la demande: **15.06.2022**
(21) Numéro de dépôt: 20306523.0
(22) Date de dépôt: 09.12.2020
(51) Int. Cl.: H01M 8/16, A61K 9/51, B01J 13/14, C12N 11/02, G01N 27/30, H01M 4/86, H01M 4/88

(54) **ELECTRODE BIO-FONCTIONNELLE DE STOCKAGE ET RELARGAGE DE COMPOSES PAR DIFFUSION LENTE**

(71) Demandeur: Centre national de la recherche scientifique, 75016 Paris (FR); Université de Lorraine, 54000 Nancy (FR); Université Grenoble Alpes, 38400 Saint-Martin-d'Hères (FR)
(72) Inventeur: COSNIER, Serge, 38920 CROLLES (FR); MC MURTRY, Stefan, 54520 LAXOU (FR); EL MAZRIA, Omar, 54280 SEICHAMPS (FR); RIAHI, Hanna, 54500 VANDOEUVRE LES NANCY (FR); NEDELLEC, Yannig, 38000 GRENOBLE (FR)
(74) Mandataire: IPAZ

(57) **Abrégé**

Electrode (1) comprenant un film de stockage (2). Le film de stockage (2) comprend une couche (3) d'un matériau support comprenant au moins une inclusion (4) dans laquelle est stocké un composé d'intérêt et une paroi (5) de matériau d'obturation perméable obturant l'au moins une inclusion. Le film de stockage (2) comprend, en outre, une couche poreuse (6) recouvrant, au moins en partie, la paroi de matériau d'obturation, au moins une partie d'une surface de la couche poreuse constituant une surface active de l'électrode.

## Description

### Domaine technique

La présente invention se rapporte aux dispositifs bio-fonctionnels. La présente invention concerne, en particulier mais non exclusivement, le domaine des dispositifs analytiques, tels que des (bio)capteurs, des dispositifs (bio)électrochimiques, tels que des (bio)-piles, et des dispositifs de relargage/libération de composés (bio)chimiques/biologiques.

### Etat de la technique antérieure

On connait dans l'état de la technique antérieure, des bio-piles dont le fonctionnement requiert l'ajout de produits (bio)chimiques, tels que par exemple des additifs, des cofacteurs ou des substrats enzymatiques. En effet, les bio-piles de l'état de la technique nécessitent que des composés chimiques, par exemple le substrat enzymatique ou microbien consommé à l'anode, soient rajoutés en continu pour que la bio-pile puisse fonctionner.

On connait également dans l'état de la technique antérieure des biocapteurs comprenant un bio-récepteur, par exemple protéique ou enzymatique. Le fonctionnement de tels biocapteurs est basé sur l'activation ou l'inhibition du bio-récepteur. L'activation du bio-récepteur permet, dans la plupart des cas, une détection spécifique d'un analyte particulier alors que l'inhibition du bio-récepteur permet, dans la plupart des cas, une détection d'un groupe de composés inhibiteurs.

On connait dans l'état de la technique antérieure, des capsules ou pilules pour le relargage/libération de composés, principalement médicamenteux, de manière contrôlée notamment dans un organisme. S'agissant des capsules, elles sont majoritairement de taille submillimétrique et destinées à être introduites directement dans un milieu biologique liquide de sorte à migrer jusqu'à une cible spécifique de l'organisme.

Un but de l'invention est notamment :
- de proposer un échantillon macroscopique pour stocker un ou plusieurs composés pour les libérer ultérieurement, et/ou
- de proposer un échantillon macroscopique pour conserver un ou plusieurs composés destinés à être libérés ultérieurement, et/ou
- de proposer un échantillon macroscopique pour libérer au moment voulu et de manière homogène, continue et contrôlée un ou des composés stockés dans l'échantillon, et/ou
- de proposer un échantillon macroscopique sous forme de film, présentant une épaisseur inférieure au millimètre, de préférence inférieure à 500 microns, qui sépare deux faces principalement planes dont la surface est supérieure au millimètre carré, de préférence supérieure au centimètre carré, apte à libérer au moment voulu et de manière homogène, continue et contrôlée un ou des composés stockés dans l'échantillon, et/ou
- de proposer un dispositif analytique destiné à être utilisé directement sur site et qui ne nécessite l'ajout d'aucun composé ou élément extérieur pour fonctionner, et/ou
- de réaliser des électrodes, en particulier des (bio)-anodes, pour (bio)-piles, destinées à être utilisées directement dans un milieu, par exemple biologique, quelconque et ne nécessitant aucun ajout de produits ou composés pour fonctionner, et/ou
- de réaliser des biocapteurs, en particulier enzymatiques, destiné à être utilisé directement sur site et ne nécessitant aucun ajout de produits ou composés pour fonctionner.

### Présentation de l'invention

A cet effet, il est proposé une électrode comprenant :
- un film de stockage comprenant :
   µ une couche d'un matériau support comprenant au moins une inclusion, l'au moins une inclusion contenant un composé d'intérêt,
   • une paroi de matériau d'obturation obturant l'au moins une inclusion, ladite paroi de matériau d'obturation étant apte à laisser transiter ou diffuser le composé d'intérêt à travers elle,
- une couche poreuse recouvrant, au moins en partie, la paroi de matériau d'obturation, au moins une partie d'une surface de la couche poreuse constituant une surface active de l'électrode.

L'au moins une inclusion peut comprendre un ou plusieurs composés d'intérêt. Le ou les composés d'intérêt peuvent être liquides ou solides, par exemple une poudre.

De préférence, la paroi de matériau d'obturation est dite perméable.

De préférence, la paroi de matériau d'obturation est apte à faire transiter ou diffuser le composé d'intérêt à travers la paroi de matériau d'obturation.

De préférence, la paroi de matériau d'obturation perméable est apte à laisser transiter, de manière contrôlée, le composé d'intérêt à travers la paroi de matériau d'obturation par exemple selon une vitesse de transit ou de diffusion souhaitée.

Une paroi de l'au moins une inclusion peut comprendre, ou peut être constituée en tout ou partie par, la paroi de matériau d'obturation.

L'au moins une inclusion peut présenter un diamètre de Féret minimal supérieur à 0,1 µm et un diamètre de Féret maximal inférieur au diamètre de l'inclusion.

De préférence, l'inclusion est entièrement comprise dans la couche de matériau support. De préférence, le film de stockage englobe ou enveloppe l'inclusion.

De préférence, la couche de matériau support et la paroi de matériau d'obturation forment un ensemble ou une couche ou un film d'un seul tenant. L'ensemble ou la couche ou le film d'un seul tenant peut constituer le film de stockage.

Le film de stockage peut être un dispositif bio-fonctionnel selon l'invention.

L'électrode peut comprendre une surface, dite de relargage ou de libération, depuis laquelle le composé d'intérêt est relargué ou libéré du film de stockage, ladite surface de relargage :
- comprend, au moins en partie, une surface de la paroi de matériau d'obturation,
- est située en regard de la couche poreuse.

La couche poreuse peut recouvrir, au moins en partie la surface de relargage.

La surface de relargage peut comprendre, au moins en partie, une des faces ou une surface du film stockage. L'une des faces ou la surface du film stockage peut correspondre à une partie de la surface totale du film stockage.

De préférence, la paroi de matériau d'obturation perméable est apte à libérer ou relarguer le composé fonctionnel par la surface de relargage.

De préférence, la paroi de matériau d'obturation perméable est apte à laisser transiter le composé d'intérêt à travers la paroi de matériau d'obturation lorsque, de préférence uniquement dans le cas où, la surface de relargage est en contact avec un milieu prédéterminé.

Dans la présente demande, il peut être entendu par paroi perméable, une paroi, un film ou une couche de matériau permettant la libre diffusion d'une ou plusieurs espèces à travers lui.

La paroi de matériau d'obturation peut être perméable du fait :
- de la propriété intrinsèque du matériau d'obturation à laisser transiter ou diffuser le composé d'intérêt, ou
- de la présence d'au moins un pore, ou au moins une perforation, dans le matériau d'obturation ; le matériau d'obturation étant imperméable, c'est à dire intrinsèquement inapte à laisser transiter ou diffuser le composé d'intérêt.

La paroi de matériau d'obturation peut être perméable, en outre, à un ou plusieurs milieux prédéterminés dans le ou lesquels l'électrode est destinée à être immergée.

De préférence, la paroi de matériau d'obturation perméable est apte à libérer ou relarguer, de manière contrôlée, le composé fonctionnel par la surface de relargage, par exemple selon une vitesse de relargage ou de libération souhaitée. De préférence, la vitesse de relargage ou de libération est égale à vitesse de transit ou de diffusion du composé d'intérêt à travers le matériau d'obturation.

De préférence, le composé d'intérêt est libéré/relargué depuis la surface de relargage dans un milieu avec lequel la surface de relargage est destinée à être mise en contact.

La couche poreuse peut comprendre un catalyseur.

Le catalyseur peut être inorganique, par exemple un complexe ou composé de coordination, ou organique, par exemple un catalyseur enzymatique.

Selon l'invention :
- le matériau d'obturation ou le matériau d'obturation et le matériau support peuvent être des matériaux conducteurs, ou
- le film de stockage peut être isolant et/ou la couche poreuse peut comprendre un matériau conducteur poreux et le catalyseur.

L'électrode peut être destinée à être utilisée comme électrode d'une pile ou d'une bio-pile, par exemple une bio-pile à combustible. L'électrode peut être destinée à être utilisée comme électrode d'une bio-pile ou comme électrode d'un biocapteur.

Le catalyseur peut être immobilisé dans et/ou sur et/ou sous le matériau conducteur poreux.

La couche poreuse peut comprendre, en outre, un matériau de piégeage perméable dans lequel est contenu le catalyseur.

Le matériau de piégeage perméable est apte à laisser transiter, de préférence de manière contrôlée, le composé d'intérêt. Le matériau de piégeage perméable est apte à laisser transiter des ions, de préférence un électrolyte ou des ions aptes constitués un électrolyte.

Le matériau de piégeage peut être un isolant électrique.

Le matériau de piégeage peut comprendre un médiateur d'oxydo-réduction, dit médiateur redox, apte à faire transiter un courant électrique dans le matériau de piégeage. Dans ce cas, la couche poreuse peut être une couche conductrice poreuse.

Le matériau de piégeage peut être un gel et/ou un polymère. De préférence, le catalyseur est immobilisé dans le matériau de piégeage.

Le matériau de piégeage peut former une couche ou un film ou peut être un ensemble de nano et/ou micro et/ou macro particules. Le film ou la couche de matériau de piégeage peut être disposée dans et/ou sur et/ou sous le matériau conducteur poreux. L'ensemble de particules formant le matériau de piégeage peut être disséminé dans la couche poreuse ou peut être amassé dans une zone de la couche poreuse.

L'au moins une inclusion peut présenter la forme d'une cavité s'étendant dans la couche du matériau support.

L'électrode peut comprendre plusieurs inclusions individuelles ayant chacune une forme oblongue ou ronde s'étendant principalement selon l'épaisseur (e) du film de stockage.

De préférence, l'épaisseur (e) du film de stockage s'étend principalement selon une direction reliant le film de stockage à la couche poreuse.

L'au moins une inclusion peut constituer au moins un réservoir individuel enveloppé, au moins en partie, dans la couche du matériau support.

Le film de stockage peut comprendre un matériau encapsulant enveloppant au moins en partie l'au moins une inclusion de sorte qu'une surface du matériau encapsulant constitue au moins une partie d'une paroi de l'au moins une inclusion.

De préférence, le matériau encapsulant constitue un film ou une couche. De préférence encore, le matériau encapsulant enveloppe uniquement en partie l'inclusion.

Le matériau encapsulant peut être constitué d'un matériau différent du matériau support. Le matériau encapsulant peut être identique au matériau support.

Le matériau encapsulant peut être identique au matériau d'obturation. De préférence, la couche de matériau support et le film de matériau encapsulant forment un ensemble ou une couche ou un film d'un seul tenant. L'ensemble, la couche ou le film d'un seul tenant formé(e) par la couche de matériau support et le film de matériau encapsulant peut constituer la couche de matériau support.

De préférence, la couche de matériau support, le film de matériau encapsulant et la paroi de matériau d'obturation forment un ensemble ou une couche ou un film d'un seul tenant. L'ensemble, la couche ou le film d'un seul tenant formé(e) par la couche de matériau support, le film de matériau encapsulant et la paroi de matériau d'obturation peut constituer le film de stockage.

De préférence, le matériau support, le matériau encapsulant et/ou le matériau d'obturation sont flexibles et/ou élastiques.

De préférence, le matériau support, le matériau encapsulant et/ou le matériau d'obturation est un matériau polymère.

La matériau d'obturation peut être différent du matériau support et/ou du matériau encapsulant.

La paroi de matériau d'obturation peut être une paroi poreuse ou semi-perméable.

Il peut être entendu par paroi poreuse une couche, un film ou une paroi comprenant au moins un pore. Le pore peut être un conduit ou un canal traversant, de part en part, la paroi dans laquelle il s'étend.

Un diamètre de pore ou un coefficient de diffusion peut être agencé pour obtenir une vitesse de relargage ou de libération du composé d'intérêt voulue. Une vitesse de relargage ou de libération du composé d'intérêt peut être fonction du milieu dans lequel est destinée à être immergée l'électrode.

Le matériau d'obturation peut être poreux ou perméable ou semi-perméable.

Le matériau d'obturation peut être imperméable et peut comprendre un ou plusieurs pores ménagés dans la paroi pour rendre la paroi de matériau d'obturation perméable.

Selon l'invention, il est également proposé une utilisation de l'électrode selon l'invention comme pile ou bio-pile ou dans une pile ou une bio-pile.

Selon l'invention, il est également proposé une utilisation de l'électrode selon l'invention comme capteur ou biocapteur ou dans un capteur ou un biocapteur.

Selon l'invention, il est également proposé une utilisation de l'électrode selon l'invention comme dispositif bio-fonctionnel ou dans un dispositif bio-fonctionnel.

Selon l'invention, il est également proposé un procédé de fabrication d'une électrode comprenant un film de stockage, ledit film de stockage comprenant au moins une inclusion, ledit procédé comprenant les étapes consistant à :
- former au moins une cavité dans une couche d'un matériau support,
- déposer un film de matériau d'obturation sur la couche de matériau support pour obturer l'au moins une cavité de sorte à former l'au moins une inclusion dans la couche de matériau support,
- former une couche poreuse sur le film de matériau d'obturation ; au moins une partie d'une surface de la couche poreuse étant apte à former une surface active d'électrode de l'électrode.

De préférence, la couche poreuse comprend le matériau conducteur poreux et le catalyseur.

Le film de matériau d'obturation peut être appliqué sur la couche de matériau support.

De préférence, l'au moins une inclusion comprend le composé d'intérêt. Le composé d'intérêt sous forme de poudre peut être destiné à être relargué ou libéré par trempage de l'électrode dans un milieu liquide. Un composé d'intérêt sous forme de poudre peut présenter l'avantage d'être relargué plus lentement qu'un composé d'intérêt stocké sous forme liquide.

Le composé d'intérêt peut être relargué ou libéré, de manière contrôlée, par exemple lorsque de l'électrode est immergée dans un liquide prédéterminé. Le composé d'intérêt peut être relargué ou libéré, de préférence uniquement, lorsque de l'électrode est immergée dans un liquide prédéterminé

L'au moins une cavité présente un diamètre de Féret minimal supérieur à 0,1 µm et un diamètre de Féret maximal inférieur au diamètre de l'inclusion.

Il peut être entendu par « film de matériau d'obturation » une pellicule solide, une bande solide ou une couche solide de matériau qui est déposée sur la couche de matériau support. De préférence, le film de matériau d'obturation est d'un seul tenant.

De préférence, chaque cavité est comprise dans la couche de matériau support.

De préférence, chaque cavité contient un seul orifice à obturer.

Chaque orifice de chaque cavité peut être obturé par la paroi de matériau d'obturation.

De préférence, subséquemment à l'étape de dépôt du film de matériau d'obturation, l'inclusion est obturée par le film de matériau d'obturation qui constitue la paroi de matériau d'obturation.

La paroi de matériau d'obturation peut être perméable.

Le procédé peut comprendre :
- préalablement à l'étape de formation de l'au moins une inclusion, une étape consistant à déposer la couche de matériau support sur une couche de substrat,
- subséquemment à l'étape de dépôt du film de matériau d'obturation, une étape consistant à dissoudre la couche de substrat de sorte à obtenir le film de stockage.

Le procédé peut comprendre, préalablement à l'étape de dépôt du film de matériau d'obturation, une étape consistant à recouvrir une paroi de l'au moins une cavité formée dans la couche de matériau support avec une couche d'un matériau encapsulant.

La paroi de l'au moins une cavité peut correspondre à la paroi de l'au moins une inclusion, à l'exception de la partie de la paroi de l'au moins une inclusion formée par le matériau d'obturation, lorsque l'étape de recouvrement de la paroi de l'au moins une cavité n'est pas mise en œuvre.

La paroi de l'au moins une cavité peut être différente de la paroi de l'au moins une inclusion lorsque l'étape de recouvrement de la paroi de l'au moins une cavité est mise en œuvre. Dans ce cas, le matériau encapsulant peut constituer la paroi de l'au moins une inclusion.

L'étape consistant à recouvrir une paroi de l'au moins une cavité peut comprendre un dépôt, de préférence isotrope, d'une couche ou d'un film de matériau encapsulant.

Le procédé peut comprendre une étape consistant à remplir l'au moins une inclusion avec un composé d'intérêt.

L'étape de remplissage de l'au moins une cavité peut être mise en œuvre préalablement à l'étape de dépôt du film de matériau d'obturation.

La mise en œuvre de l'étape de remplissage préalablement à l'étape de dépôt convient, en particulier, aux composés d'intérêt solides mais peut également convenir aux composés d'intérêt liquides.

L'étape de remplissage de l'au moins une occlusion peut être mise en œuvre, subséquemment à l'étape de dépôt du film de matériau d'obturation, en injectant le composé d'intérêt ou un précurseur du composé d'intérêt au moyen d'un capillaire ou d'une aiguille traversant la paroi de matériau d'obturation.

La mise en œuvre de l'étape de remplissage subséquemment à l'étape de dépôt convient, en particulier, aux composés d'intérêt liquides mais peut également convenir aux composés d'intérêt solides.

Subséquemment à l'étape de dépôt, le procédé peut comprendre une étape consistant à rendre perméable la paroi de matériau d'obturation par perforation mécanique ou par traitement ultrasons, chimique, optique ou plasma de la paroi d'obturation.

Le procédé peut comprendre une étape consistant à réaliser une ou plusieurs perforations de la paroi de matériau d'obturation ; la ou les perforations présentant un diamètre supérieur à 10 nm.

L'étape de remplissage peut être mise en œuvre concomitamment à l'étape de perforation lorsque l'étape de remplissage est réalisée par injection du composé d'intérêt ou du précurseur du composé d'intérêt au moyen d'un capillaire ou d'une aiguille traversant la paroi de matériau d'obturation.

De préférence, l'étape de perforation est mise en œuvre préalablement à l'étape consistant à former une couche poreuse sur le film de matériau d'obturation.

Le diamètre de la ou des perforations peut s'étendre selon une direction perpendiculaire à l'épaisseur de la paroi de matériau d'obturation. L'épaisseur de la paroi de matériau d'obturation peut s'étendre selon la direction reliant le film de stockage à la couche poreuse.

L'étape de dépôt de la couche poreuse peut comprendre les sous-étapes consistant à :
- déposer un matériau conducteur poreux sur au moins une partie de la paroi de matériau d'obturation, puis
- immobiliser un catalyseur (8) dans et/ou sur et/ou sur le matériau conducteur poreux ;
la couche poreuse comprenant le matériau conducteur poreux et le catalyseur.

De préférence, le matériau conducteur poreux est déposé sur au moins une partie, de préférence sur l'ensemble, de la surface de relargage.

Le procédé peut comprendre une étape consistant à piéger et/ou immobiliser le catalyseur dans un matériau de piégeage.

Subséquemment ou concomitamment à l'étape de piégeage du catalyseur dans le matériau de piégeage, le procédé peut comprendre une étape consistant à déposer le matériau de piégeage contenant le catalyseur dans et/ou sur et/ou sous le matériau conducteur poreux.

Le procédé selon l'invention convient à la mise en œuvre de l'électrode selon l'invention. L'électrode selon l'invention est, de préférence, mise en œuvre par le procédé selon l'invention. De préférence, le procédé selon l'invention est spécialement conçu pour mettre en œuvre l'électrode selon l'invention. Ainsi, toute caractéristique du procédé selon l'invention peut être intégrée dans l'électrode selon l'invention et inversement.

### Description des figures

D'autres avantages et particularités de l'invention apparaîtront à la lecture de la description détaillée de mises en œuvre et de modes de réalisation nullement limitatifs, et des dessins annexés suivants :
[Fig. 1] les figures 1A, 1B et 1C sont des représentations schématiques des étapes du procédé de fabrication de dispositifs bio-fonctionnels selon l'invention,
[Fig. 2] les figures 2A, 2B, 2C et 2D sont des représentations schématiques des étapes du procédé de fabrication de dispositifs bio-fonctionnels selon l'invention,
[Fig. 3] les figures 3A et 3B sont des représentations schématiques d'électrodes selon l'invention,
[Fig. 4] la figure 4 est un graphique illustrant l'évolution de la variation de la concentration molaire en bleu de méthylène, libéré via la surface de relargage, en fonction du temps pour deux dispositifs bio-fonctionnels selon l'invention : un film de stockage et une électrode,

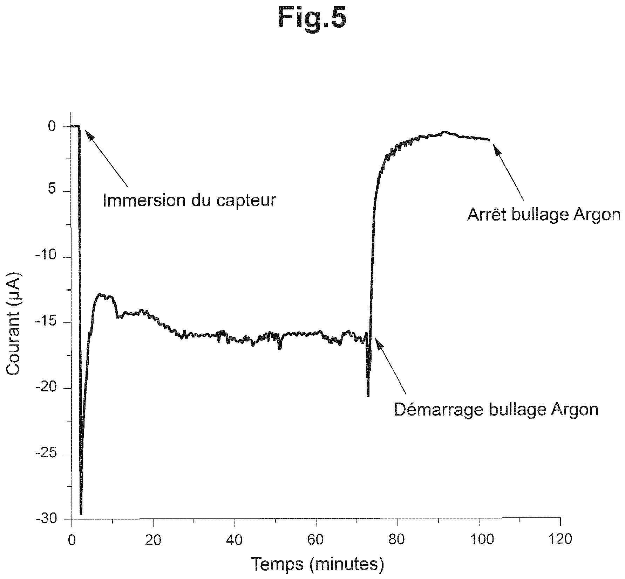

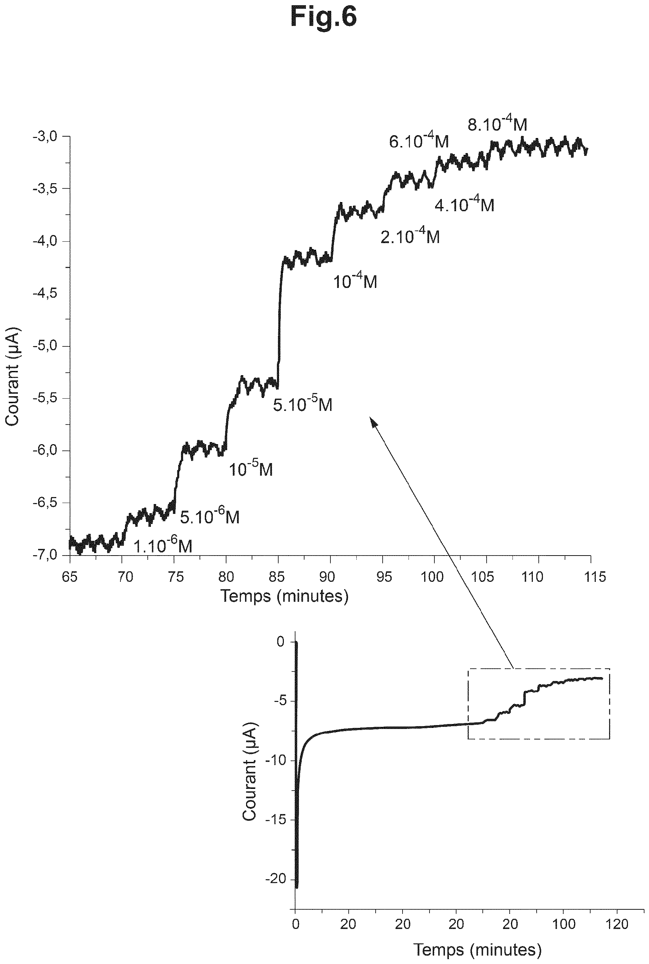

[Fig. 5] la figure 5 est un graphique représentant l'évolution du courant cathodique, généré par la réduction d'orthoquinone en catéchol par l'électrode selon l'invention, en fonction du temps et de la quantité d'oxygène dissous en solution ; l'électrode, comprenant de la tyrosinase comme catalyseur et du catéchol comme composé d'intérêt, est utilisée comme biocapteur pour détecter l'oxygène dissous en solution, [Fig. 6] la figure 6 est un graphique représentant l'évolution du courant cathodique, généré par la réduction d'orthoquinone en catéchol par l'électrode selon l'invention, en fonction du temps et de la quantité d'acide benzoïque en solution ; l'électrode, comprenant de la tyrosinase comme catalyseur et du catéchol comme composé d'intérêt, est utilisée comme biocapteur pour détecter de l'acide benzoïque en solution.

### Description des modes de réalisation

Les modes de réalisation décrits ci-après étant nullement limitatifs, on pourra notamment considérer des variantes de l'invention ne comprenant qu'une sélection de caractéristiques décrites, isolées des autres caractéristiques décrites (même si cette sélection est isolée au sein d'une phrase comprenant ces autres caractéristiques), si cette sélection de caractéristiques est suffisante pour conférer un avantage technique ou pour différencier l'invention par rapport à l'état de la technique antérieure. Cette sélection comprend au moins une caractéristique, de préférence fonctionnelle sans détails structurels, ou avec seulement une partie des détails structurels si cette partie uniquement est suffisante pour conférer un avantage technique ou pour différencier l'invention par rapport à l'état de la technique antérieure.

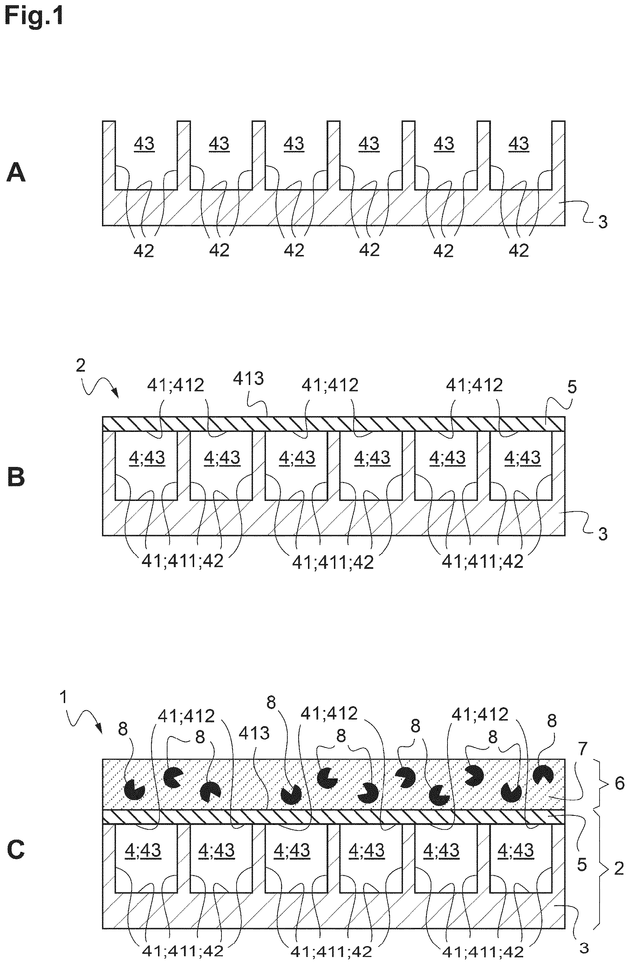

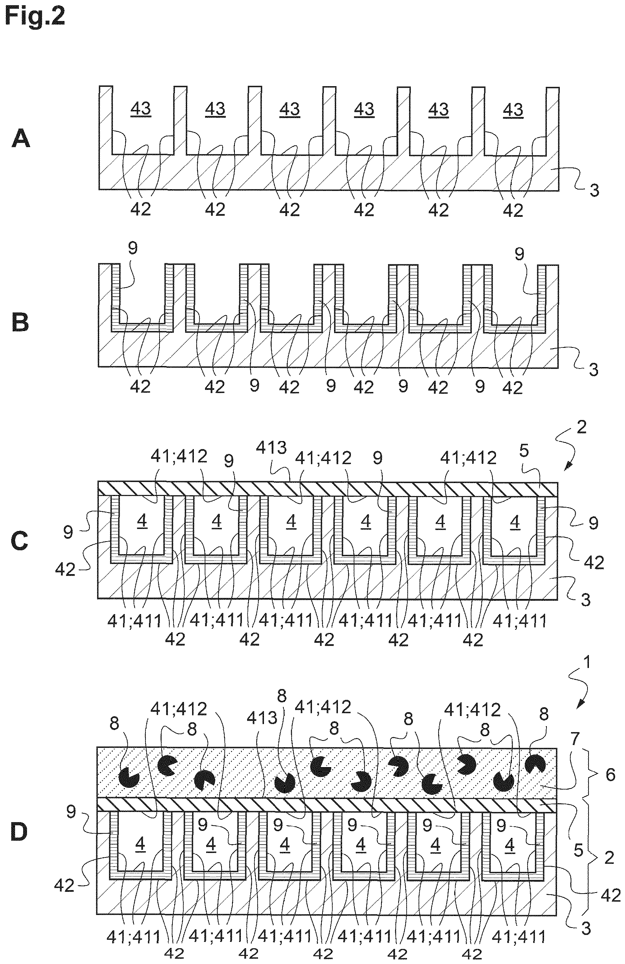

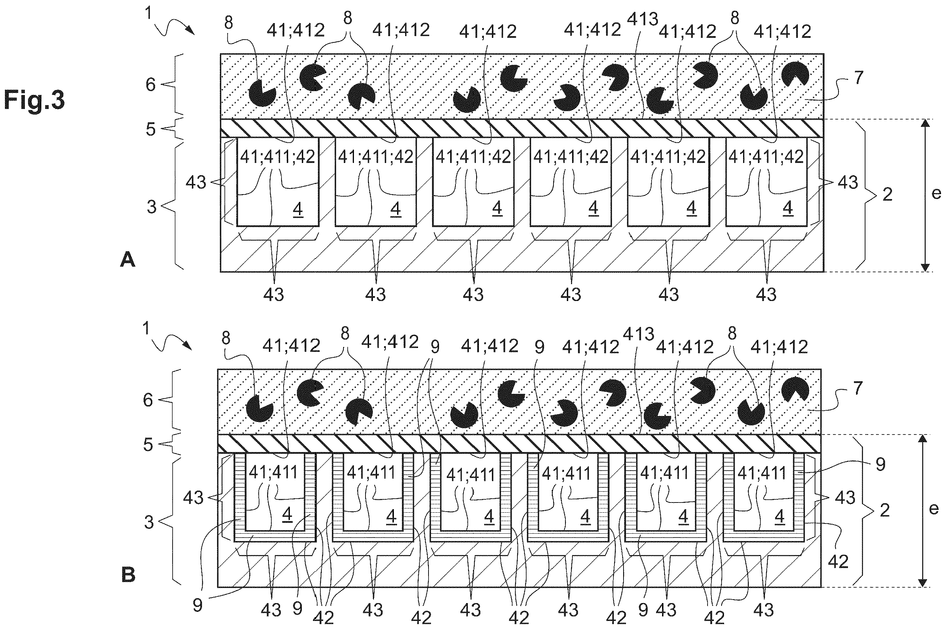

En référence aux FIGURES 1 et 2, il est illustré un mode de réalisation d'un procédé de fabrication d'un dispositif bio-fonctionnel 1. En référence aux FIGURES 3, il est illustré un mode de réalisation d'une électrode 1. L'épaisseur du film de stockage (2) est typiquement comprise entre 50 microns et plusieurs centimètres. De manière préférée, l'épaisseur du film de stockage (2) est comprise entre 500 µm et 5 mm. Le film de stockage 2 comprend au moins une inclusion 4.

En référence aux FIGURES 1 et 2, le procédé comprend l'étape consistant à former au moins une cavité 43 dans la couche 3 de matériau support. Le matériau support est un polymère. A titre d'exemple non limitatif, le matériau support est un matériau souple (déformable) ou rigide (non déformable) tel que le silicium, l'oxyde de zinc, le nitrure d'aluminium, l'oxyde de silicium, l'oxyde d'aluminium, les polymères, les matériaux plastiques et les métaux. De préférence, le matériau support est en polyurethane (PU) ou en poly-méthacrylate de méthyle (PMMA). L'au moins une cavité 43 présente un diamètre de Féret compris entre 0,05 µm et 5 mm, de préférence entre 0,1 µm et 1 mm.

Le procédé de fabrication comprend, en outre, l'étape consistant à déposer le film 5 de matériau d'obturation sur la couche 3 de matériau support pour obturer l'au moins une cavité 43 de sorte à former l'au moins une inclusion 4 dans la couche 3 de matériau support. A titre d'exemple non limitatif, le matériau d'obturation est en polyuréthane (PU) et donc imperméable selon le mode de réalisation. L'épaisseur du film 5 de matériau d'obturation est typiquement comprise entre 1 µm et 1 cm, de préférence entre 25 µm et 400 µm. Le matériau d'obturation peut être choisi parmi les matériaux métalliques, les matériaux semi-conducteurs, les matériaux piézoélectriques, les matériaux isolants et les polymères tels que les résines époxydes et les résines biocompatibles. A titre d'exemple non limitatif, le matériau d'obturation peut être du PMMA, du polyurethane, des polymeres biocompatibles, du métal, tel que de l'or par exemple. Le matériau d'obturation est apte à résister au solvant utilisé durant l'étape de dissolution de la résine.

Le procédé comprend une étape consistant à remplir l'au moins une inclusion 4 avec un composé d'intérêt qui dépend du type d'application visée. L'étape de remplissage de l'au moins une cavité 43 est mise en œuvre préalablement à l'étape de dépôt du film 5 de matériau d'obturation. L'étape de remplissage consiste à immerger le film de stockage 2, préalablement à l'étape de dépôt du film 5 de matériau d'obturation, dans une solution de composé d'intérêt. De manière alternative, l'étape de remplissage peut consister à déverser, préalablement à l'étape de dépôt du film 5 de matériau d'obturation, dans l'inclusion 4 le composé d'intérêt se présentant, par exemple, sous forme de poudre.

Le procédé comprend une étape consistant à réaliser une ou plusieurs perforations de la paroi 5 de matériau d'obturation. Selon un mode de réalisation, la ou les perforations sont réalisées au moyen d'un capillaire ou d'une aiguille par perforation mécaniquement de la paroi (5) de matériau d'obturation. La ou les perforations présentent un diamètre supérieur à 10 nm. A titre d'exemple non limitatif, le diamètre de la ou des perforations est compris entre 100 µm et le diamètre de Féret de la cavité 43 selon l'application visée ainsi que selon le type et l'état solide ou liquide du composé d'intérêt.

Selon un mode réalisation non limitatif, le procédé comprend l'étape consistant à former une couche poreuse 6 sur le film 5 de matériau d'obturation. L'épaisseur de la couche poreuse 6 est typiquement comprise entre 400 nm et 20 µm. De préférence, l'épaisseur de la couche poreuse 6 est typiquement comprise entre 1 et 5 µm. Au moins une partie d'une surface de la couche poreuse 6 est apte à former une surface active de l'électrode 1.

L'étape de dépôt de la couche poreuse 6 comprend l'étape consistant à déposer un matériau conducteur poreux 7, des nanotubes de carbone (NTC) 7 selon le mode de réalisation, sur au moins une partie de la paroi 5 de matériau d'obturation, sur l'ensemble de paroi 5 d'obturation selon le mode de réalisation. L'épaisseur de NTC 7 est typiquement identique à l'épaisseur de la couche poreuse 6. L'étape de dépôt de la couche poreuse 6 comprend, en outre, l'étape consistant à immobiliser un catalyseur 8 dans et/ou sur les NTC 7. La couche poreuse 6 comprend donc les NTC 7 et le catalyseur 8.

Selon le mode de réalisation non limitatif, et en référence à la FIGURE 2B, le procédé comprend, préalablement à l'étape de dépôt du film 5 de matériau d'obturation, une étape consistant à recouvrir une paroi 42 de l'au moins une cavité 43, formée dans la couche 3 de matériau support, avec une couche 9 d'un matériau encapsulant en PU. Dans ce cas, la paroi 41 de la couche 9 du matériau encapsulant constitue la paroi 41 de l'inclusion 4 à l'exception de la partie 412 de la paroi 41 de l'au moins une inclusion 4 formée par le matériau d'obturation. L'épaisseur de la couche 9 de matériau encapsulant est typiquement supérieure à 10 nm, de préférence comprise entre 30 µm et la moitié du diamètre de Féret de la cavité 43. Le matériau encapsulant peut être choisi parmi les matériaux métalliques, les matériaux semi-conducteurs, les matériaux piézoélectriques, les matériaux isolants et les polymères tels que les résines époxydes et les résines biocompatibles. De préférence, le matériau encapsulant est apte à résister au solvant utilisé dans l'étape de dissolution de la résine. A titre d'exemple non limitatif, le matériau encapsulant peut être du PMMA, du polyurethane, des polymeres biocompatibles, du métal, tel que de l'or par exemple.

En référence aux FIGURES 3, il est décrit une électrode 1 comprenant un film de stockage 2 et une couche poreuse 6. Le film de stockage 2 comprend une couche 3 d'un matériau support comprenant au moins une inclusion 4. L'au moins une inclusion 4 contient au moins un composé d'intérêt qui diffère selon le type d'application visée. Le film de stockage 2 comprend, en outre, une paroi 5 de matériau d'obturation perméable obturant l'au moins une inclusion 4. La paroi 5 de matériau d'obturation perméable est une paroi poreuse 5.

Selon le mode de réalisation, l'électrode 1 est obtenue directement par mise en œuvre du procédé précédemment décrit.

L'au moins une inclusion 4 constitue au moins un réservoir individuel 4 enveloppé, au moins en partie, dans la couche 3 du matériau support.

Selon le mode de réalisation, l'électrode 1 comprend plusieurs inclusions individuelles 4 ayant chacune une forme oblongue et s'étendent principalement selon l'épaisseur e du film de stockage 2.

La couche poreuse 6 recouvre, au moins en partie, entièrement selon le mode de réalisation, la paroi 5 de matériau d'obturation. Au moins une partie d'une surface de la couche poreuse 6 constitue une surface active de l'électrode 1.

Selon le mode de réalisation, la paroi 41 de l'au moins une inclusion 4, à l'exception de la partie 412 de la paroi 41 de l'au moins une inclusion 4 formée par le matériau d'obturation, est une surface du matériau support.

L'électrode 1 comprend une surface 413 de relargage depuis laquelle le composé d'intérêt est libéré du film de stockage 2. La surface 413 de relargage comprend, au moins en partie une surface de la paroi 5 de matériau d'obturation. La surface 413 de relargage est située en regard de la couche poreuse 6. Selon le mode de réalisation, les surfaces de la paroi 5 de matériau d'obturation situées au regard de la couche poreuse 6 et face aux parties 412 de la paroi 41 du matériau d'obturation formant des parties 412 de parois 41 des inclusions 4 constituent la surface de relargage 413.

Selon un mode de réalisation non limitatif, le matériau support est identique au matériau d'obturation. Le film de stockage 2 est donc isolant selon le mode de réalisation.

La couche poreuse 6 comprend un catalyseur 8, un électro-catalyseur 8 selon le mode de réalisation. La couche poreuse 6 comprend, en outre, un matériau conducteur poreux 7, des nanotubes de carbone (NTC) 7 selon le mode de réalisation. Selon le mode de réalisation, au moins une partie des NTC 7 constitue la surface active de l'électrode 1.

Selon un mode de réalisation non limitatif illustré sur la FIGURE 3B, le film de stockage 2 comprend un matériau encapsulant 9 enveloppant au moins en partie l'au moins une inclusion 4 de sorte qu'une surface 411 du matériau encapsulant constitue au moins une partie de la paroi 41 de l'au moins une inclusion 4.

Dans ce cas, la paroi de l'au moins une inclusion 4, à l'exception de la partie 412 de la paroi 41 de l'au moins une inclusion 4 formée par le matériau d'obturation, est une surface du film 9 ou de la couche 9 de matériau encapsulant enveloppant l'inclusion 4.

Selon ce mode de réalisation non limitatif, le matériau encapsulant 9 est du PU.

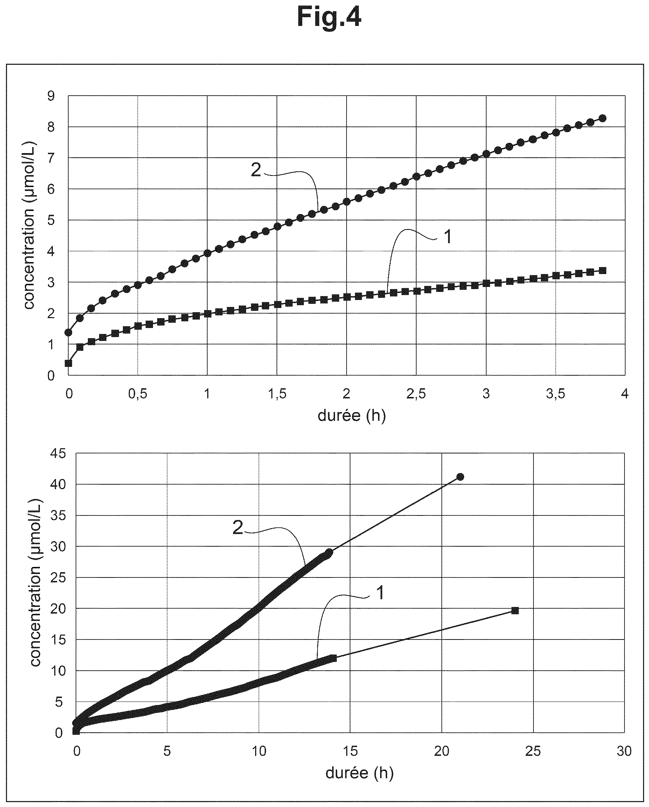

En référence à la FIGURE 4, il est présenté un graphique illustrant la variation de la concentration molaire d'un composé d'intérêt dans une solution dans laquelle une électrode 1 et un film de stockage 2 selon l'invention est immergée. Le composé d'intérêt est du bleu de méthylène qui est libéré par le film de stockage 2 dans la solution en fonction du temps. La vitesse de relargage et la régularité du relargage ont été évaluées pour le film de stockage 2 selon l'invention et pour l'électrode 1 selon l'invention.

Le bleu de méthylène est stocké sous forme de poudre dans les inclusions 4. L'étape de remplissage des inclusions 4 a été mise en œuvre par écoulement dans les inclusions 4, sous l'effet de la gravité, du bleu de méthylène sous forme de poudre. L'étape de perforation a été mise en œuvre en réalisant, pour chaque inclusion 4, une seule perforation de la partie 412 de la paroi 41 de chaque inclusion 4 formée par le matériau d'obturation au moyen d'une aiguille d'un diamètre typiquement égal à 100 µm. L'étape de perforation a été réalisée au moyen d'un microscope optique et d'un système de guidage mécanique de l'aiguille.

Concernant l'électrode 1, l'étape de dépôt des NTC 7 a été réalisée par dépôt d'une solution (dispersion) de NTC 7 sur la paroi 5 de matériau d'obturation d'un film de stockage 2 selon l'invention. La solution a été laissée pendant 120 minutes sous vide sur la paroi 5 puis rincée. La concentration de NTC 7 dans la solution de NTC 7 est de 5 mg/ml. Le solvant est de l'éthylène glycol. Les solvants tels que le N,N-diméthylformamide (DMF) ou le N-méthylpyrrolidone (NMP) ont été proscrits car ils dénaturent et détériorent la plupart des polymères et donc le film de stockage 2 selon le mode de réalisation. Après rinçage du film de stockage 2, les NTC 7 adhérent fortement au film de stockage 2, l'électrode 1 ainsi formée est prête pour utilisation.

Le film de stockage 5 a été immergé dans un volume de 2,5 ml de solution aqueuse. La concentration en bleu de méthylène dissous dans la solution aqueuse a été mesurée par spectrométrie UV/visible. Sur les FIGURES 4a et 4b est représentée l'évolution de la concentration totale en bleu de méthylène en micromoles par litres (µmol/l) dans la solution aqueuse en fonction du temps en heures (h). Il est observé une augmentation linéaire de la concentration au cours du temps. Cela indique une vitesse de relargage constante. La vitesse de relargage du film de stockage 2 est comprise entre 1,5 et 4 µmol/l/h. La vitesse de relargage de l'électrode 1 est comprise entre 0,5 et 0,8 µmol/l/h.

En référence aux FIGURES 5 et 6, il est présenté une utilisation comme biocapteur de l'électrode telle que présentée aux FIGURES 3. Selon le mode de réalisation en question, la couche poreuse 6 comprend des NTC 7, un catalyseur enzymatique 8, la tyrosinase 8, et une argile, la laponite. Le composé d'intérêt stocké dans l'inclusion 4, qui est libéré progressivement lors de l'utilisation de l'électrode 1, est du catéchol. L'étape de remplissage a été mise en œuvre par écoulement dans les inclusions 4, sous l'effet de la gravité, du catéchol sous forme de poudre. L'étape de perforation a été mise en œuvre en réalisant, pour chacune des deux inclusions 4, une seule perforation de la partie 412 de la paroi 41 de chaque inclusion 4 formée par le matériau d'obturation au moyen d'une aiguille d'un diamètre typiquement égal à 100 µm.

En pratique, le procédé de mise en œuvre de la couche poreuse 6 comprend une dispersion et une délamination de la laponite en milieu aqueux. La laponite est présente à une concentration massique de 1 mg/ml. La tyrosinase 8 est ensuite mélangée à la solution aqueuse de laponite à une concentration de 10 mg/ml. La solution comprenant le mélange laponite-tyrosinase est ensuite déposé sur l'électrode 1, comprenant un film de NTC 7 sur la paroi 5 de matériau d'obturation du film de stockage 2. Les NTC 7 ont été préalablement déposés sur l'électrode 1, conformément à ce qui est décrit ci-dessus. Le volume de solution de laponite-tyrosinase déposé est tel qu'environ 300 µg de tyrosinase 8 et 30 µg de laponite repose sur le film de NTC 7. Après dépôt de la solution comprenant le mélange laponite-tyrosinase, il est procédé à l'évaporation du solvant, c'est à dire l'eau, sous vide. L'électrode 1, est ensuite placée sous vapeur de glutaraldéhyde pendant 45 min afin de réticuler le mélange laponite-tyrosinase. Avant utilisation de l'électrode 1, la laponite est gélifiée par trempage de l'électrode 1, dans une solution tampon hydrogénophosphate/di-hydrogénophosphate pendant 20 min.

En référence aux FIGURES 5 et 6, il est illustré l'évolution du courant cathodique généré par l'électrode utilisée comme biocapteur 1. Pour la mise en œuvre de l'expérimentation, l'électrode 1, est immergée dans une solution tampon hydrogénophosphate/di-hydrogénophosphate, dite solution tampon. En présence d'oxygène et de catéchol, la tyrosinase 8 oxyde le catéchol, qui est libéré au niveau de la surface de relargage 413, en ortho-quinone. En appliquant une tension de -0,2 Volts, par rapport au potentiel de référence de l'électrode au calomel saturée (ECS), à l'électrode 1, on observe l'apparition d'un courant cathodique qui est généré à l'électrode 1.

En référence à la FIGURE 5, il est illustré la possibilité de détecter la présence ou l'absence d'oxygène dissous en solution au moyen de l'électrode 1. Au moyen d'un étalonnage préalable, il est également possible de quantifier la quantité d'oxygène dissous en solution. Le courant cathodique généré à l'électrode 1, se stabilise à environ 15 µampères (µA) après une vingtaine de minutes. Après 70 minutes, il est procédé à un bullage d'argon dans la solution tampon. Ceci a pour effet de retirer la quasi-totalité de l'oxygène dissous dans la solution tampon et donc de diminuer de manière substantielle, voire d'arrêter, l'activité de la tyrosinase 8 et donc l'oxydation du catéchol par la tyrosinase 8. La quantité d'orthoquinone dans la couche poreuse 6 diminue donc également lorsque la concentration en oxygène dissous diminue. On observe alors une chute du courant cathodique à une valeur proche de 0 Ampères. La chute du courant est proportionnelle à la diminution de la concentration en oxygène en solution. Cela démontre que le courant cathodique généré à l'électrode 1 provient effectivement de la réduction de l'orthoquinone en catéchol par l'électrode 1.

En référence à la FIGURE 6, il est illustré la possibilité de détecter la présence d'inhibiteurs de la tyrosinase 8, ici l'acide benzoïque, au moyen de l'électrode 1. Cet inhibiteur peut être un polluant. Au moyen d'un étalonnage préalable, il est également possible de quantifier la quantité d'acide benzoïque en solution. Le courant cathodique généré à l'électrode 1 se stabilise à environ 7,5 µA après une vingtaine de minutes. Après 65 minutes, il est procédé à neuf injections successives de quantités connues d'acide benzoïque dans la solution tampon. Ceci a pour effet d'inhiber progressivement la tyrosinase 8 qui voit donc son activité diminuer lorsque la concentration en acide benzoïque augmente. La quantité d'orthoquinone dans la couche poreuse 6 diminue donc également lorsque la concentration en acide benzoïque augmente. Il est observé une diminution par palier du courant cathodique généré à l'électrode 1 pour chacune des injections d'acide benzoïque. Le courant diminue depuis 7 µA, la valeur du courant cathodique avant la première injection réalisée à la 65^{eme} minute pour une concentration en acide benzoïque de 0 Molaire (M), jusqu'à 3 µA à la 112^{eme} minute pour une concentration totale en acide benzoïque de 8.10⁻⁴ M. La chute du courant est proportionnelle à la quantité d'inhibiteur en solution. Cela démontre que le courant cathodique généré à l'électrode 1 provient effectivement de la réduction de l'orthoquinone en catéchol par l'électrode 1.

Ainsi, dans des variantes combinables entre elles des modes de réalisation précédemment décrits :
- le matériau d'obturation est un matériau perméable, et/ou
- la paroi 5 de matériau d'obturation est une paroi semi-perméable 5, et/ou
- le matériau d'obturation ou le matériau d'obturation et le matériau support sont des matériaux conducteurs, et/ou
- l'épaisseur du matériau conducteur poreux 7 est égale à l'épaisseur de la couche poreuse 6 lorsque le catalyseur 8 et le matériau conducteur poreux 7 sont déposés concomitamment, et/ou
- la couche de substrat est en poly-acétate de vinyle (PVA) ou du PMMA, et/ou
- le matériau support est différent du matériau d'obturation, et/ou
- l'électrode 1 comprend plusieurs inclusions individuelles 4 ayant chacune une forme ronde, et/ou
- le procédé comprend :
   - préalablement à l'étape de formation de l'au moins une inclusion 4, une étape consistant à déposer la couche 3 de matériau support sur une couche de substrat,
   - subséquemment à l'étape de dépôt du film 5 de matériau d'obturation, une étape consistant à dissoudre la couche de substrat de sorte à obtenir le film de stockage 2, et/ou
- l'étape de remplissage de l'au moins une occlusion 4 est mise en œuvre, subséquemment à l'étape de dépôt du film 5 de matériau d'obturation, en injectant le composé d'intérêt ou un précurseur du composé d'intérêt au moyen d'un capillaire ou d'une aiguille traversant la paroi 5 de matériau d'obturation, et/ou
- lorsque le procédé ne comprend pas d'étape de recouvrement de la cavité 43 par une couche 9 de matériau encapsulant, la paroi 42 de la cavité 43 constitue la paroi 41 de l'inclusion 4, et/ou
- la perforation de la partie 412 de la paroi 41 de chaque inclusion 4 formée par le matériau d'obturation est réalisée au moyen d'une aiguille d'un diamètre supérieur à 100 µm, et/ou
- l'étape de perforation est mise en œuvre par une technique ne générant pas d'effet joule local pour ne pas altérer la paroi de matériau d'obturation ni introduire d'imprécision quant à la taille des pores créés, et/ou
- l'étape de perforation est mise en œuvre par laser femto ou par abrasion ultrasonique.

De plus, les différentes caractéristiques, formes, variantes et modes de réalisation de l'invention peuvent être associés les uns avec les autres selon diverses combinaisons dans la mesure où ils ne sont pas incompatibles ou exclusifs les uns des autres.

## Revendications

1. Electrode (1) comprenant :
- un film de stockage (2) comprenant :
• une couche (3) d'un matériau support comprenant au moins une inclusion (4), l'au moins une inclusion contenant un composé d'intérêt,
• une paroi (5) de matériau d'obturation obturant l'au moins une inclusion, ladite paroi de matériau d'obturation étant apte à laisser transiter ou diffuser le composé d'intérêt à travers elle,
- une couche poreuse (6) recouvrant, au moins en partie, la paroi de matériau d'obturation, au moins une partie d'une surface de la couche poreuse constituant une surface active de l'électrode.

2. Electrode (1) selon la revendication 1, comprenant une surface (413), dite de relargage ou de libération, depuis laquelle le composé d'intérêt est relargué ou libéré du film de stockage (2), ladite surface de relargage :
- comprend, au moins en partie, une surface de la paroi (5) de matériau d'obturation,
- est située en regard de la couche poreuse (6).

3. Electrode (1) selon la revendication 1 ou 2, dans laquelle la couche poreuse (6) comprend un catalyseur (8).

4. Electrode (1) selon l'une quelconque des revendications précédentes, dans laquelle :
- le matériau d'obturation ou le matériau d'obturation et le matériau support sont des matériaux conducteurs, ou
- le film de stockage (2) est isolant et la couche poreuse (6)comprend un matériau conducteur poreux (7) et le catalyseur (8).

5. Electrode (1) selon l'une quelconque des revendications précédentes, comprenant plusieurs inclusions individuelles (4) ayant chacune une forme oblongue ou ronde s'étendant principalement selon l'épaisseur (e) du film de stockage (2).

6. Electrode (1) selon l'une quelconque des revendications précédentes, dans laquelle l'au moins une inclusion (4) constitue au moins un réservoir individuel enveloppé, au moins en partie, dans la couche (3) du matériau support.

7. Electrode (1) selon l'une quelconque des revendications précédentes, dans laquelle le film de stockage (2) comprend un matériau encapsulant (9) enveloppant au moins en partie l'au moins une inclusion (4) de sorte qu'une surface (411) du matériau encapsulant constitue au moins une partie d'une paroi (41) de l'au moins une inclusion (4).

8. Electrode (1) selon l'une quelconque des revendications précédentes, dans laquelle la paroi (5) de matériau d'obturation est une paroi poreuse ou semi-perméable.

9. Utilisation de l'électrode (1) selon l'une quelconque des revendications 1 à 8, dans une pile ou dans un capteur.

10. Procédé de fabrication d'une électrode (1) comprenant un film de stockage (2), ledit film de stockage comprenant au moins une inclusion (4), ledit procédé comprenant les étapes consistant à :
- former au moins une cavité (43) dans une couche (3) d'un matériau support,
- déposer un film (5) de matériau d'obturation sur la couche (3) de matériau support pour obturer l'au moins une cavité de sorte à former l'au moins une inclusion dans la couche de matériau support,
- former une couche poreuse (6) sur le film (5) de matériau d'obturation ; au moins une partie d'une surface de la couche poreuse étant apte à former une surface active d'électrode (1).

11. Procédé selon la revendication 10, comprenant :
- préalablement à l'étape de formation de l'au moins une inclusion (4), une étape consistant à déposer la couche (3) de matériau support sur une couche de substrat,
- subséquemment à l'étape de dépôt du film (5) de matériau d'obturation, une étape consistant à dissoudre la couche de substrat de sorte à obtenir le film de stockage (2).

12. Procédé selon la revendication 10 ou 11, comprenant, préalablement à l'étape de dépôt du film (5) de matériau d'obturation, une étape consistant à recouvrir une paroi (41, 42) de l'au moins une cavité (43) formée dans la couche (3) de matériau support avec une couche (9) d'un matériau encapsulant.

13. Procédé selon l'une quelconque des revendications 10 à 12, comprenant une étape consistant à remplir l'au moins une inclusion (4) avec un composé d'intérêt.

14. Procédé selon la revendication précédente, dans lequel l'étape de remplissage de l'au moins une cavité (43) est mise en œuvre préalablement à l'étape de dépôt du film (5) de matériau d'obturation.

15. Procédé selon la revendication 13, dans lequel l'étape de remplissage de l'au moins une occlusion (4) est mise en œuvre, subséquemment à l'étape de dépôt du film (5) de matériau d'obturation, en injectant le composé d'intérêt ou un précurseur du composé d'intérêt au moyen d'un capillaire ou d'une aiguille traversant une paroi (5) de matériau d'obturation.

16. Procédé selon l'une quelconque des revendications 10 à 15, comprenant une étape consistant à réaliser une ou plusieurs perforations de la paroi (5) de matériau d'obturation ; la ou les perforations présentant un diamètre supérieur à 10 nm.

17. Procédé selon l'une quelconque des revendications 10 à 16, comprenant l'étape consistant à former une couche poreuse (6) sur le film (5) de matériau d'obturation ; au moins une partie d'une surface de la couche poreuse étant apte à former une surface active de l'électrode (1).

18. Procédé selon l'une quelconque des revendications 10 à 17, dans lequel l'étape de dépôt de la couche poreuse (6) comprend les sous-étapes consistant à :
- déposer un matériau conducteur poreux (7) sur au moins une partie de la paroi (5) de matériau d'obturation, puis
- immobiliser un catalyseur (8) dans et/ou sur et/ou sous le matériau conducteur poreux ;
la couche poreuse comprenant le matériau conducteur poreux et le catalyseur.
